# EUROPEAN PATENT APPLICATION

(11) **EP 1 602 931 A1**
(43) Date of publication of application: **07.12.2005**
(21) Application number: 04076659.4
(22) Date of filing: 03.06.2004
(51) Int. Cl.: G01N 33/68, A61K 38/10, A61P 19/02

(54) **SR-A antagonists**

(71) Applicant: Universiteit Leiden, 2312 AV Leiden (NL)
(72) Inventor: Biessen, Erik Anna Leonardus, 2331 RX Leiden (NL); van Berkel, Theodorus Josephus Cornelis, 2024 DP Haarlem (NL)
(74) Representative: Winckels, Johannes Hubertus F.

(57) **Abstract**

The present invention relates to a method for obtaining an antagonist of scavenger receptor SR-A, said method comprising providing a peptide, testing binding of said peptide to the ligand binding site of scavenger receptor SR-A and selecting said peptide in case said peptide is capable of binding to said ligand binding site of SR-A, thereby obtaining the antagonist. The invention further relates to a peptide capable of binding to the ligand binding site of an SR-A receptor, and to the use of such a peptide or a conjugate thereof as a medicament for treating atherosclerosis or inflammation. Moreover, the invention relates to a method for producing a medicament, comprising producing a plurality of different peptides, testing binding of these peptides to the ligand binding site of SR-A, selecting a peptide which binds to said ligand binding site of SR-A and manufacturing a medicament comprising the thus selected peptide.

## Description

### TECHNICAL FIELD

The invention relates to methods for obtaining receptor antagonists suitable for the treatment and prevention of diseases associated with activity of said receptor. In particular, the present invention relates to methods for obtaining SR-A antagonists suitable for the treatment and prevention of atherosclerosis. The present invention relates to compositions suitable for use in such treatments and in particular to non-immunogenic compositions comprising therapeutic peptides.

### BACKGROUND OF THE INVENTION

Atherosclerosis is a condition in which deposits of fatty substances, cholesterol, cellular waste products, calcium and other substances build up in the inner lining of an artery. This build-up is called plaque. It usually affects large and medium-sized arteries. Some hardening of arteries often occurs when people grow older. Plaques can grow large enough to significantly reduce the blood's flow through an artery. But most of the damage occurs when they become fragile and rupture. Plaques that rupture cause blood clots to form that can block blood flow or break off and travel to another part of the body. If either happens and blocks a blood vessel that feeds the heart, it causes a heart attack. If it blocks a blood vessel that feeds the brain, it causes a stroke.

Although atherosclerosis is a complex process, it appears that the following contributes to lesion formation: under the influence of disturbed hemodynamics and elevated serum cholesterol levels, the endothelium gradually becomes dysfunctional and starts to express adhesion molecules; the smooth muscle cells in the artery wall begin to proliferate; inflammatory cells are recruited to the subendothelium; and lipids, or fats, accumulate in and around the cells. It is believed that the process begins when cells on the surface of the artery wall become damaged, and begin to interact with circulating blood monocytes. The monocytes adhere to the wall, migrate underneath the surface layer, and change into scavenger cells, or macrophages. These macrophages accumulate subendothelial lipids and transdifferentiate into foam cells which are typified by the presence of included fat globules.

The complex nature of the atherosclerotic diseases implies that medicaments used in its treatment can be aimed at one of various processes involved in the disease. One of the most promising routes is believed to reside in inhibiting the process of foam cell formation. The foam cell develops as a result of uptake of chemically modified lipoproteins by the macrophage through a specific receptor exposed on its cell surface.

The modified lipoprotein receptor was first described by Brown and Goldstein in 1979 (Goldstein *et al*., 1979). Because of its ability to bind a broad range of negatively charged polyanions, such as modified low-density lipoprotein (LDL), the receptor was referred to as scavenger receptor-AI (SR-AI). At present, besides SR-AI more than six classes of scavenger receptors have been identified (Krieger, 1997; Greaves *et al*., 1998; Terpstra *et al*., 2000), of which CD36 (SR class B), CD68 (SR class D), lectin-like oxidized LDL receptor (LOX-1, SR class E), scavenger receptors expressed by endothelial cells (SREC, SR class F), and scavenger receptors for phosphatidyl serine and oxidized lipoprotein (SR-PSOX, SR class G) were all shown to be involved in foam cell formation. The first to be cloned was bovine SR-A, a trimeric transmembrane glycoprotein consisting of 6 distinct domains (Freeman *et al*., 1990; Kodama *et al*., 1990; Rohrer *et al*., 1990). SR-A exists in one of three isoforms (SR-AI, SR-AIl and SRA-111) which differ in the cysteine-rich domain and/or collagen-like domain responsible for ligand binding (Doi *et al*., 1993; Acton *et al*., 1993; Tanaka *et al*., 1993; Tanaka *et al*., 1996). SR-A has a very broad ligand specificity and not only recognizes chemically modified lipoproteins (e.g. OxLDL, MDA-LDL, and AcLDL) but also polyribonucleotides (e.g. polyinosinic acid (poly 1) and polyguanylic acid (poly G)); polysaccharides (e.g. dextran sulphate and fucoidin) and anionic macromolecules like polyvinyl sulphate and lipopolysaccharide (LPS) (Nagelkerke *et al*., 1983; Nishikawa *et al*., 1990; Hampton *et al*., 1991; Ashkenas *et al*., 1993; Resnick *et al*., 1993; Zhang *et al*., 1993; Dunne *et al*., 1994; Greenberg *et al*., 1996; Van Berkel *et al*., 1998).

SR-A is mainly expressed in resident macrophages of various organs including liver Kupffer cells (Hughes *et al*., 1995; Naito *et al*., 1991; Naito *et al*., 1992) but also by endothelial cells (Blomhoff *et al*., 1984; Daugherty *et al*., 1997; Geng and Hansson, 1995) and smooth muscle cells (Pitas, 1990). Interestingly, SR-AI was found to be considerably overexpressed in atherosclerotic lesions *in vivo* (Matsumoto *et al*., 1990; Yla-Hertualla *et al*., 1991; Bickel and Freeman, 1992; Luoma *et al*., 1994; Geng *et al*., 1995; Li *et al*., 1995; Mietus-Snyder *et al*., 1997). This has fueled speculation as to a potential role of SR-AI in lesion formation (reviews see Linton and Fazio, 2001).

Indeed scavenger receptors were found to mediate the accumulation of modified LDL by macrophages and other plaque cells leading to the formation of lipid laden foam cells, as was observed after overexpression of SR-A by Chinese hamster ovary cells and macrophages (Freeman *et al*., 1991). The importance of SR-A in atherosclerosis was shown by Suzuki et al (Suzuki *et al*., 1997) who demonstrated that lesion formation was 58% lower in SR-A-deficient ApoE-/- mice as compared to SR-A containing ApoE-/- mice. Subsequent studies suggested that SR-AI significantly contributes to atherosclerotic lesion formation and atherogenesis. (Babaev *et al*., 2000). In addition to its contribution to atherosclerosis, SR-A has been shown to be important in host defense (Krieger, 1997; Pearson, 1996), adhesion and cell-cell interaction (El Koury *et al*., 1994; Fraser *et al*., 1993; Hughes *et al*., 1994; Van Velzen *et al*., 1999; Yokota *et al*., 1998). The abundant expression of SR-AI on macrophage rich sites of inflammation and atherosclerotic lesions and the fact that this receptor mediates the efficient endocytosis of bound substrates indicates that SR-AI may not only be a interesting target for therapeutic intervention in atherosclerosis and inflammation but also for drug delivery and targeted imaging approaches.

Given the complex chemical nature of the macromolecular substrates for SR-AI it is not surprising that ligand design for this receptor has not been very successful and has only resulted in a single report on a synthetic rather unselective SR-A antagonist (Lysko *et al*., 1999).

An alternative route is to provide antibodies against SR-A. In fact, antibodies against SR-AI are known. However these have as a disadvantage that they may elicit an immunogenic reaction.

Yet another possibility for obtaining SR-A receptor antagonists is to use cells that expose the SR-A receptor on their surface, or even the isolated SR-A receptors itself, as targets in a screening assay for substances that bind to the receptor. A disadvantage of such methods is that the binding of the substance may occur to a part of the cell instead of to the receptor, or to a part of the receptor that is itself not involved in binding the natural ligand of the receptor. For instance, the substance selected may have bound to a part of the receptor that is unrelated or remote to the actual ligand binding site. This will result in the identification of a substance that is capable of binding to the receptor, but that is not capable of blocking the receptor. Therefore, such methods do not necessarily result in the identification of an antagonist. Thus, the methods of the prior art have not produced effective antagonists for the SR-A receptor.

### SUMMARY OF THE INVENTION

The present inventors have now found a new approach in the design of SR-AI antagonists involving the use of phage display on a synthetic receptor containing the ligand-binding pocket of SR-AI. A novel short peptide ligand was identified, which appeared to be specific for SR-AI.

In one aspect the invention now provides a for obtaining an antagonist of scavenger receptor SR-A, said method comprising providing a peptide, testing binding of said peptide to the ligand binding site of scavenger receptor SR-A and selecting said peptide in case said peptide is capable of binding to said ligand binding site of SR-A, thereby obtaining the antagonist. In a preferred embodiment said peptide is further capable of blocking said ligand binding site of SR-A. In another a preferred embodiment said SR-A receptor is the SR-AI receptor. In yet another preferred embodiment, said ligand binding site is a synthetic ligand binding site.

In still another preferred embodiment, said ligand binding site encompassing the C-terminal Lys cluster (320-340) of the collagen-like domain of the macrophage scavenger receptor whereby the protein preferably is in a triple stranded configuration.

In still another preferred embodiment, said peptide is provided in the form of a phage display library.

In another aspect, the invention provides a peptide obtainable by a method of the invention.

In another aspect, the invention provides a peptide capable of binding to the ligand binding site of an SR-A receptor, preferably also capable of at least inhibiting the binding of natural or synthetic SR-A ligands to the SR-A receptor. In a preferred embodiment, said SR-A receptor is the SR-AI receptor. In another preferred embodiment, said ligand binding site is a synthetic ligand binding site. In yet another preferred embodiment, said ligand binding site is the ligand binding pocket.

A peptide of the present invention comprises a sequence according to the formula (I):

X₁-X₂-RFLRCWSD-X₃-P-X₄

in which
X₁ is optionally present and may be a carboxylic acid; or one or more D- or L-amino acids or analogues or mimetics thereof; or an anti-inflammatory drug; or a medicament for treating atherosclerosis; or a detectable label;
X₂ is a D- or L-amino acid or a peptide, preferably selected from the group consisting of the peptide (Leu or Ala or Ile)-Ser-(Leu or Ala or Ile)-(Glu or Asp), the peptide Ser-(Leu or Ala or Ile)-(Glu or Asp), the peptide (Leu or Ala or Ile)-(Glu or Asp), and of the amino acids Glu and Asp;
X₃ is a D- or L-amino acid, preferably selected from Ala, Ser, Leu, Ile, Cys and Thr, more preferably selected from Ala and Ser;
X₄ is optionally present and may be a D- or L-amino acid, preferably selected from Ala, Ser, Leu, Ile, Cys and Thr, more preferably selected from Ala and Ser, or an analogue or mimetic thereof; or an anti-inflammatory drug; or a medicament for treating atherosclerosis; or a detectable label.

As stated, X₁ en X₄ are optionally present and may advantageously be introduced to optimize the affinity of the core peptide by introducing additional interaction possibilities with any residual groups in the receptor binding site. Preferably the position defined by X₄ is used to introduce a functional group (i.e. an anti-inflammatory drug, a medicament for treating atherosclerosis or a detectable label).

Also the peptide of the invention may be a truncated peptide of a peptide according to formula I, provided that said truncated peptide is capable of binding to SR-AI.

Preferred peptides of the invention comprise a sequence selected from the group consisting of SEQ ID NO: 1 - SEQ ID NO: 5. In yet other preferred embodiments said peptide is conjugated to an anti-inflammatory drug, a medicament for treating atherosclerosis or a detectable label.

In another aspect, the invention provides a peptide or a peptide-conjugate according to the invention for use as a medicament.

Other aspects of the invention include the use of a peptide or a peptide-conjugate according to the invention, for the manufacture of a medicament for the treatment of *atheroselerosis* and/or i*nflammation*. Yet other uses include the use of a peptide according to the invention, as an SR-A antagonist.

The present invention also relates to the use of a peptide or a peptide-conjugate according to the invention, as a drug-delivery vehicle, and to the use of such a peptide or a peptide-conjugate in a method for targeted imaging of atherosclerotic lesions.

In another aspect, the invention provides a pharmaceutical composition comprising a peptide or peptide-conjugate according to the invention and a pharmaceutically acceptable carrier or diluent. In a preferred embodiment thereof said composition is in the form of an intravenously or subcutaneously injectable fluid.

In another aspect, the invention provides a method of imaging atherosclerotic lesions or diagnosing rupture-sensitive atherosclerotic plaques, comprising the step of administering to a subject in need thereof a peptide-conjugate according to the invention wherein said conjugate comprises a label, followed by the step of *in vivo* detecting said peptide.

In another aspect, the invention provides a method for producing a medicament or pharmaceutical composition, comprising producing a plurality of different peptides, testing binding of these peptides to the ligand binding site of SR-A, selecting a peptide which binds to said ligand binding site of SR-A and manufacturing a medicament or pharmaceutical composition comprising the thus selected peptide. In a preferred embodiment, said medicament or pharmaceutical composition is for the treatment of atherosclerosis. In another preferred embodiment, said medicament or pharmaceutical composition comprises the selected peptide together with a pharmaceutically acceptable carrier or diluent. In yet another preferred embodiment said peptide is conjugated to an anti-atherosclerotic drug.

In another aspect, the invention provides a pharmaceutical composition obtainable by a method for producing a medicament or pharmaceutical composition according to the invention.

In another aspect, the invention provides a method of treating atherosclerosis comprising administering to a patient in need thereof a therapeutically effective amount of the pharmaceutical composition according to the invention.

### DESCRIPTION OF THE DRAWINGS

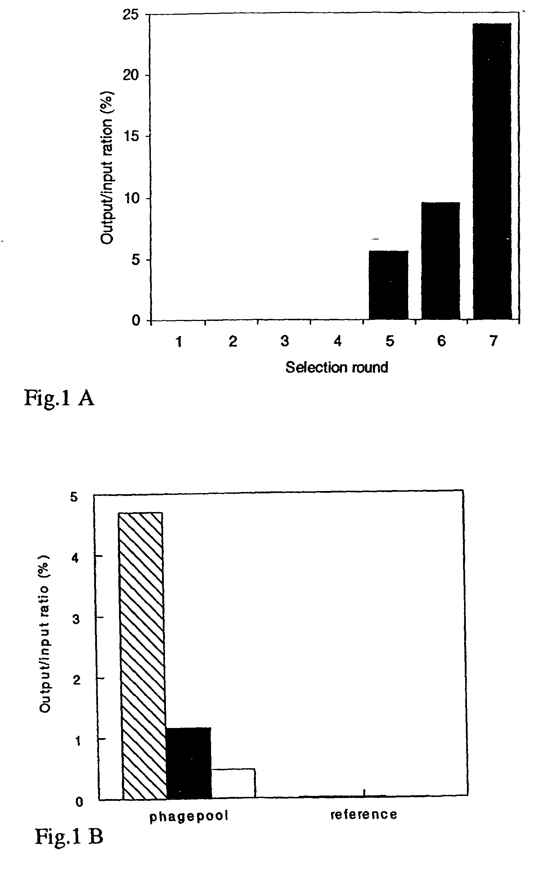

Figure 1 shows the selection and specificity of SR-AI binding phage. Panel (A) shows the selection on immobilized, biotinylated, synthetic, truncated SR-AI, comprising the ligand binding pocket using the pComb3 X15 phage displayed peptide library resulted in a 918-fold enrichment of SR-AI binding phage in the 5th round of selection. Panel (B) shows the enriched phage pool specifically bound to biotinylated SR-AI peptide (hatched bars) but not to streptavidin immobilized biotin (black/filled bars) or collagen S (open bars). As a reference, non-specific phage pool showed little binding to SR-AI. Values are expressed as ratio of output/input ratio and represent means of duplicate experiments.

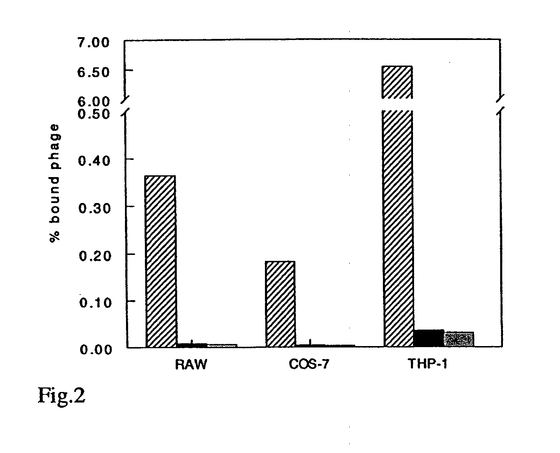

Figure 2 shows the cell binding of enriched phage pool. Enriched phage (hatched bars) but not the parental library (WT; black bars) and the controlled non-specific phage library (open bars) displayed avid binding to human THP-1 cells, moderate binding to murine RAW cells and poor binding to COS-7 cells. Values are expressed as output/input ratio and are means of two independent experiments.

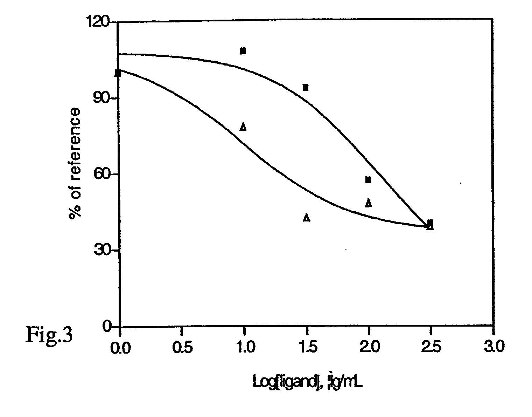

Figure 3 shows the competition of the selective phage binding to immobilized, biotinylated, synthetic, truncated SR-AI by established SR-AI inhibitors. Binding of selective phage pool to biotinylated SR-AI was determined in the presence of Suc-HSA (■) or polyI (Δ). The IC₅₀ values, as calculated from the competition curves were 66 µg/ml and 10.5 µg/ml, respectively. Values are expressed as a percentage of total phage binding in the absence of the inhibitors and represent means of two independent experiments.

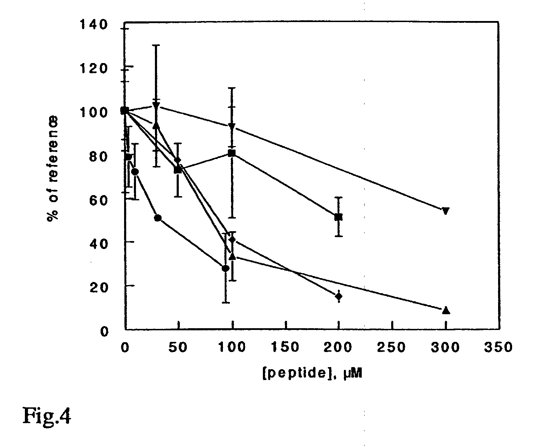

Figure 4 shows the competition of selective phage binding to biotinylated SR-AI peptide by synthetic peptides. Binding of the enriched phage pool was determined in the presence of 0-300 µm of LSLERFLRCWSDAPA (●), LERFLRCWSDAPA (▲), RFLRCWSDAPA (◆), LRCWSDAPA (■), CWSDAPA (▼). Values are expressed as a percentage of total binding in the absence of peptide and represent means of three independent experiments ±SD. The IC₅₀ values as calculated from the displacement curves were 29.1 µM, 72.3 µM, 80.9 µM, uncalculated, and 217 µM, respectively.

### DEFINITIONS

The term "antagonist" is used herein in its art-recognized form and specifically refers to a chemical substance that interferes with the physiological action of another chemical substance, especially by binding to and/or blocking a receptor.

The term "receptor" as used herein, is a molecular structure or site on the surface or interior of a cell that binds physiologically with substances such as hormones, antigens, drugs, or neurotransmitters.

The term "receptor ligand" as used herein, is an ion, a molecule, or a molecular group, preferably a peptide that binds to a receptor to form a larger complex therewith.

The term "binding site" as used herein refers to the specific reactive parts or domains of a macromolecule, such as e.g. a receptor, that directly participate in its specific combination with another molecule, such as e.g. the ligand, which is termed herein the "ligand binding site". Such a part of the receptor typically comprises a functional three-dimensional architecture and may be a singular continuous binding site or may be a discontinuous multi-domain site. As used herein the terms "binding site" and "ligand binding site" are meant to indicate an isolated molecular structure, e.g. a synthetic peptide, that comprises or, preferably, consists of that part of the receptor that represents the actual site that is involved (specifically) in the binding of the ligand, to the exclusion of those parts of the receptor that are capable of binding a peptide when such binding does not interfere with the binding of the natural ligand to that receptor, and preferably in a configuration essentially functionally similar to the configuration of the binding site in the native and properly folded form of the receptor.

The term "functionally similar" means that the isolated ligand binding site, in its function as a binding domain, must perform the same function, provide the same ligand binding utility and be capable of contributing to the same process as the native receptor. While it need not contain every feature of the native receptor, the principal features must be present.

The term "synthetic" as used herein refers to a chemical compound or material produced by synthesis, especially not of natural origin.

The term "amino acid", as used herein refers to an organic compound containing an amino group (NH₂), a carboxylic acid group (COOH), and any of various side groups, especially any of the 20 compounds that have the basic formula NH₂CHRCOOH, including synthetic analogues comprising an amino group and a carboxylic acid function, herein also referred to as an amino acid analogue or mimetic thereof

The terms "peptide", "polypeptide" and "protein" are used interchangeably herein to refer to any of various natural or synthetic compounds containing two or more amino acids linked by the carboxyl group of one amino acid to the amino group of another, in particular to a polymer of amino acid residues. The terms thus apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "peptide", "polypeptide" and "protein" are also inclusive of modifications including, but not limited to, glycosylation, lipid attachment, sulphation, gamma-carboxylation of glutamic acid residues, hydroxylation and ADP-ribosylation. It will be appreciated, as is well known and as noted above, that polypeptides are not always entirely linear. For instance, polypeptides may be branched as a result of ubiquitination, and they may be circular, with or without branching, generally as a result of posttranslation events, including natural processing event and events brought about by human manipulation which do not occur naturally. Circular, branched and branched circular polypeptides may be synthesized by non-translation natural process and by entirely synthetic methods, as well. Further, this invention contemplates the use of both the methionine-containing and the methionine-less amino terminal variants of the protein of the invention

The term "therapeutically effective amount" as used herein refers to an amount of a therapeutic agent to treat, ameliorate, or prevent a desired disease or condition, or to exhibit a detectable therapeutic or prophylactic effect. The precise effective amount needed for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation.

A "derivative", "analog" or "analogue" is defined herein as a peptide that is subjected to a (bio)chemical modification (e.g. organo-chemical or enzymatical). Such derivatising may comprise the substitution of certain chemical groups to the peptide, thereby retaining the character of the compound. Peptide derivatizations are generally known in the art. The derivatives and analogues maintain the biological activity of the peptide and act in a comparable way, but may provide advantages to the molecule such as longer half-life, resistance to degradation or an increased activity. In particular derivatives of peptides according to the present invention include synthetic amino acid analogues (natural or synthetic). Amino acid analogues are chemical substances having similar molecular structure and function with normal amino acids. Examples include aliphatic amino acids, single ring aromatic amino acids, polycyclic aromatic amino acids, Single Ring heterocyclic amino acids, polycyclic heteronuclear amino acids, amino and alkylamino containing side chains, carboxyl, carboxamide, and carboxyl ester group containing side chains, guanidine and amidine containing side chains, hydroxy and alkoxy containing side chains, mercapto and alkylmercapto containing side chains, other heteroatom containing side chains, alpha-imino acids, non-α amino acids (beta-amino acids, etc.), des-carboxy and des-amino acids. The derivatives include functional equivalents, such as modified peptides, peptide analogues, or peptidomimetics. Peptidomimetics, in their broadest scope, are compounds which are in their functional structure more or less similar to a peptide, but which may also contain non-peptidic bonds in the backbone, or *D-amino* acids. In general, peptidomimetics serve as substitutes for native peptides in the interaction with receptors and enzymes (Pharmaceutical Biotechnology, Ed. D. J. A. Crommelin and R. D. Sindelar, Harwood Academic Publishers, 1997, p. 138). Pseudopeptides, a class of peptidomimetics, are compounds containing amide bond isosteres instead of amide bonds (*ibid*., pp. 137-140). Peptides of the invention also include salts of peptides or functional equivalents, such as pharmaceutically acceptable acid- or base addition salts. They also include multimers of peptides or funtional equivalents. Techniques for selecting appropriate analogues or mimetics of naturally occurring amino acids are well-known to those skilled in the art. For example, the pseudopeptide approach aims at achieving a higher chemical or enzymatic stability of a peptide while retaining its bioactivity. Peptide bonds which undergo rapid degradation *in vivo* are replaced by other functional groups to create bioisosteres or amide bond surrogates. Examples of bioisosteric groups are N-methyl amide, thioester, thioamide, ketomethylene, methyleneamino, retro-inverse amide, methylenethio, and methyleneoxy groups (ibid., pp. 138-139). An approach to improve the selectivity of a peptide is to introduce conformational constraints to the backbone, which decreases the number of potential receptor or enzyme interactions. The constraints are most often achieved by the introduction of carbon-carbon double bonds (olefinic analogues) and ring structures (*ibid*.). A further method to identify analogues which mimic the functional structure of an amino acid such as cysteine (C), methionine (M), or valine (V) is computer-aided modeling. Preferred mimetic structures possess a side chain with similar charge or electronegativity, hydrophobicity and spatial orientation to said amino acids.

A "conjugate" is defined herein as a chemical compound that has been formed by the joining of two or more compounds, either directly or via a linker.

"Subject" as used herein includes, but is not limited to, mammals, including, e.g., a human, a non-human primate, a mouse, a pig, a cow, a goat, a cat, a rabbit, a rat, a guinea pig, a hamster, a horse, a monkey, a sheep, or other non-human mammal; and non-mammal animals, including, e.g., a non-mammalian vertebrate, such as a bird (e.g., a chicken or duck) or a fish, and an invertebrate.

### DETAILED DESCRIPTION OF THE INVENTION

A method for detecting an SR-A antagonist according to the invention comprises the step of providing a peptide and a step of testing the binding of said peptide to the ligand binding site of an SR-A receptor.

A peptide as used herein may be any peptide as defined hereinabove. Preferably, the peptide used in a method of the invention is provided as part of a peptide library, such a peptide library comprising a plurality of defined and different peptides. Most preferably, the peptide used in a method of the invention is provided as part of a phage display library.

Phage display is *an in vitro* selection technique in which a peptide or protein is genetically fused to a coat protein of a bacteriophage, resulting in display of the fused protein on the exterior of the phage virion, while the DNA encoding the fusion resides within the virion. By cloning large numbers of DNA sequences into the phage, display libraries are produced with a repertoire of many billions of unique displayed proteins. This physical linkage between the displayed protein and the DNA encoding it allows screening of vast numbers of variants of the protein, each linked to its corresponding DNA sequence, by a *simple in vitro* selection procedure called "biopanning." In its simplest form, biopanning is carried out by incubating the pool of phage-displayed variants with a target of interest, i.c. the ligand binding site of the SR-A receptor, that has been immobilized on a plate or bead, washing away unbound phage, and eluting specifically bound phage by disrupting the binding interactions between the phage and target. The eluted phage is then amplified *in vivo* and the process repeated, resulting in stepwise enrichment of the phage pool in favour of the tightest binding sequences. Generally, after 3 rounds of selection/amplification, individual clones are characterized by DNA sequencing. The main advantage of phage display over other technologies is the ease with which one can screen large numbers of clones or clonal variations comprised in the fully randomized peptide libraries. Using phage display, greater than 10⁸ different displayed sequences can easily be screened, and since the selected phage pool can be amplified by propagation in for instance *E. coli,* multiple rounds of selection can be carried out to iteratively select for the tightest binding sequences.

A method for detecting an SR-A antagonist according to the invention comprises the step of testing the binding of a peptide to the ligand binding site of an SR-A receptor. Testing of the binding may for instance occur by contacting the peptide with the said ligand binding site and allowing binding to occur between the peptide and the ligand binding site of the receptor. If binding occurs, the peptide will form a complex with the ligand binding site, which complex may then be detected to indicate the presence of binding between the peptide and the ligand binding site of an SR-A receptor.

The binding may for instance be indicated by the resistance to separation of the binding pairs, for instance by resistance to the removal by washing off one of the binding pairs. Alternatively, binding may for instance be indicated by an increased mass of the combined components, or by any other means known to the person skilled in the art for establishing the binding between a peptide and a ligand binding site of a receptor.

Very convenient methods for assessing the binding between a peptide and the ligand binding site of an SR-A receptor are those that can be performed by for instance an ELISA test, wherein the ligand binding site of the SR-A receptor is immobilized in a test-well, after which the peptide is added to the well and the binding between the two is assessed by ELISA.

When using phage-display libraries, i.e. wherein said peptide is phage-displayed as a fusion protein on the exterior of a phage virion, assessment of the binding between a peptide comprised in said library and the ligand binding site of an SR-A receptor can be performed by affinity selection or "biopanning". A simple method for biopanning involves coating a plate with the target and incubating the library on the plate to allow phage displaying a complementary protein to the target to bind. Non-binding phage are then washed away and those that are bound are eluted. Infection of bacteria with the binding phage results in phage amplification. Successive rounds of biopanning enrich the pool of phage, with clones that specifically bind the target. DNA sequencing of the phage genome determines the amino acid sequence of the proteins binding to the target. Panning may for instance be performed by using direct coating methods (wherein the panning is performed against a polystyrene plate coated with the target), or alternatively by using indirect methods, for instance by using liquid suspended beads with target molecules coated thereon. The methods as described herein are well within reach of the skilled person.

The SR-A receptor, the ligand binding site of which is used in a method of the present invention, may be an SRAI, SR-AII or SR-AIII receptor, and is preferably the SR-AI receptor, as this receptor is indicated to be involved in atherosclerosis. The ligand binding site of these receptors is essentially used in the present invention.

In order to detect an SR-A receptor antagonist rather than a mere SR-A binding peptide, the present invention is aimed at using the ligand binding site of said SR-A receptor, rather than the cells possessing/exposing on their surface the SR-A receptor, or the isolated SR-A receptors itself to assess the binding of a peptide thereto. A disadvantage of using cells possessing and/or exposing on their surface the SR-A receptor, or of using the isolated SR-A receptors itself is that the binding may occur to a part of the cell or receptor that is not *per se* involved in binding the natural ligand of the receptor. For instance, the peptide selected may have bound to a part of the receptor that is unrelated, spatially removed or separate from the actual ligand binding site. In case the selected peptide is to have antagonistic effect, it should at least inhibit the binding of the natural ligand or, preferably, compete with the natural ligand for the binding site on the receptor. The peptide can even prevent the binding of the natural ligand altogether thereby effectively blocking the normal physiological consequences of ligand-receptor binding.

It is known that poly-anions with a small negative charge are suitable ligands for the SR-A. In the case of polynucleotides (DNA or RNA), it is known that quadruple helices exhibit better ligand performance than single stranded polynucleotides and that the polynucleotides should be longer than 8-10 nucleotides in length. Further, oligo-G and oligo-C are recognized by the receptor, whereas oligo-A and oligo-T are not. Known ligands for the SR-A are very different from the peptides of the present invention.

A very effective way of ascertaining the binding between a test peptide and the ligand binding site of a receptor is to prepare said ligand binding site as a synthetic ligand binding site and testing the binding of the peptide thereto. The ligand binding site is preferably the actual binding pocket of the receptor, which may for instance be prepared by synthesizing the ligand binding motif and providing conditions suitable for allowing the motif to fold in the conformation corresponding to the binding pocket of the receptor. Particular reference is made herein to Suzuki *et al*., in Biochemistry 38(6):1751-1756 (1999), wherein the preparation of a synthetic alpha-helical coiled coil domain of the macrophage scavenger receptor is reported. The triple-stranded alpha -helical coiled coil domain of the macrophage scavenger receptor is the ligand binding site of the receptor. Such a synthetic ligand binding site is advantageously used in embodiments of the method of the present invention for detecting SR-A receptor antagonists. The methods described in Suzuki *et al*., (1999) may be applied to provide synthetic ligand binding sites for SR-AI, SR-AII or SR-AIII receptors or for other scavenging receptors and may in general provide teaching to obtain synthetic ligand binding sites for other receptors.

In more preferred embodiments of aspects of the present invention testing of the binding of a peptide to the ligand binding site of an SR-A receptor is accompanied by testing of the capacity of the selected peptide to block the ligand binding site of an SR-A receptor. Blocking the ligand binding site of an SR-A receptor is referred herein as blocking and/or inhibiting the binding of the natural ligands of the receptor to their respective binding site. The capacity of the selected peptide to block the ligand binding site can easily be assessed by performing a binding competition experiment between the peptide and the natural ligand. Such experiments are well known to the skilled artisan and are exemplified in the Examples described herein below.

The ligand binding site as used in this invention encompasses the C-terminal Lys cluster (320-340) of the collagen-like domain of the macrophage scavenger receptor which is critical for binding and which should preferably be configured as a triple stranded protein..

According to the present invention, a method is provided with which peptides capable of binding to the SR-A receptor may be found that are potentially effective as antagonists for the natural SR-A receptor ligands. The fact that the peptides bind to the ligand binding site of the receptor means that these peptides have the capacity to compete with the natural receptor ligand for the binding to the receptor. Accordingly, it is an advantage of the method of the present invention that a method is provided by which potential receptor antagonists are discovered more easily and more effectively than before, because the natural receptor will also bind to the ligand binding site of the receptor and binding of a peptide thereto may prevent the natural ligand from binding.

The present invention provides a peptide detectable by a method of the invention as described herein above.

The present invention also provides a peptide capable of binding to the ligand binding site, preferably a synthetic ligand binding site, of an SR-A receptor, preferably the SR-AI receptor.

The peptides of the invention are capable of binding to the SR-A receptor and are preferably capable of at least inhibiting the binding of natural or synthetic SR-A ligands, such as poly I or succinylated human serum albumin (suc-HAS). Preferably, the peptides of the invention are capable of completely preventing or blocking the binding of natural or synthetic ligands to SR-A.

The present invention now provides for a large number of suitable peptides that can be used as SR-A antagonists. In general, such peptides comprise a sequence according to the formula (I):

X₁-X₂-Arg-Phe-Leu-Arg-Cys-Trp-Ser-Asp-X₃-Pro-X₄

hereinafter also referred to as

X₁-X₂-RFLRCWSD-X₃-P-X₄

in which
X₁ is optionally present and may be a carboxylic acid; or one or more D- or L-amino acids or analogues or mimetics thereof; or an anti-inflammatory drug; or a medicament for treating atherosclerosis; or a detectable label. The carboxylic acid may be a synthetic or natural carboxylic acid such as the C-10 to C-18 fatty acids such as citric acid, glycolic acid, tartaric acid or lactic acid; an unsaturated acid, such as caproic acid or oleic acid; a branched carboxylic acid such as naphthenic acid of molecular weight of from 200 to 500; an aromatic carboxylic acid such as benzoic or salicylic acid, or any other carboxylic acid;
X₂ is a D- or L-amino acid or a peptide, preferably X₂ is selected from the group consisting of the peptide (Leu or Ala or Ile)-Ser-(Leu or Ala or Ile)-(Glu or Asp), the peptide Ser-(Leu or Ala or Ile)-(Glu or Asp), the dipeptide (Leu or Ala or Ile)-(Glu or Asp), and the amino acids Glu and Asp, wherein the symbols indicate the amino acid in line with IUPAC nomenclature and symbolism for amino acids and peptides;
X₃ is a D- or L-amino acid, preferably selected from Ala, Ser, Leu, Ile, Cys and Thr, more preferably selected from Ala and Ser;
X₄ is optionally present and may be a D- or L-amino acid, preferably selected from Ala, Ser, Leu, Ile, Cys and Thr, more preferably selected from Ala and Ser, or an analogue or mimetic thereof; or an anti-inflammatory drug; or a medicament for treating atherosclerosis; or a detectable label, and R, F, L, C, W, S, D and P are the symbols indicating the amino acids Arg, Phe, Leu, Cys, Trp, Ser, Asp and Pro, respectively, according to IUPAC nomenclature and symbolism for amino acids.

It should be understood that the D- or L-amino acids of X₂ and/or X₃ may also take the form of analogues or mimetics of the corresponding amino acid.

In a preferred embodiment the invention provides a peptide comprising the amino acid sequence of SEQ ID NO: 1 (LSLERFLRCWSDAPA), wherein E represents the amino acid glutamic acid and wherein A represents the amino acid alanine. Thus, in this preferred embodiment X₂ in formula I is represented by the peptide LSLE (Leu-Ser-Leu-Glu), and X₃ and X₄ are both represented by the amino acid alanine.

In an alternative preferred embodiment, a peptide of the invention comprises the amino acid sequence of SEQ ID NO: 2 (LSLERFLRCWSDSPR).

Yet in another preferred embodiment the peptide of the invention comprises the amino acids that represent the consensus of SEQ ID NO: 1 and SEQ ID NO: 2, which consensus is represented by the amino acid sequence of SEQ ID NO: 3 (LSLERFLRCWSD) or truncated peptides thereof. A particularly preferred truncated peptide of SEQ ID NO: 3 is the peptide corresponding to the amino acid sequence of SEQ ID NO: 4 (LSLERFL). The minimal motif for binding to the synthetic SR-AI receptor is believed to be SEQ ID NO: 5 (LERFL), which is yet another preferred embodiment of a truncated peptide of SEQ ID NO: 3.

The invention further provides a peptide comprising a sequence selected from the group consisting of SEQ ID NO: 1, SEQ ID NO: 2, SEQ ID NO: 3, SEQ ID NO: 4 and SEQ ID NO: 5. The peptides corresponding to the amino acid sequences of SEQ ID NO: 1-5 are capable of binding to the synthetic ligand binding site of SR-AI as described in the Examples below.

The invention further provides a peptide essentially consisting of a sequence selected from the group consisting of: the peptides according to the formula (I) X₁-X₂-RFLRCWS-X₃-P-X₄ as defined above; the peptides of SEQ ID NO: 3 and truncated forms thereof; and derivatives of these peptides. These peptides may be used as antagonists of natural ligands of SR-AI.

The peptides of the present invention may have a length of between 4 and 250 amino acid residues. In preferred embodiments, the peptide of the invention is a peptide of from 5 to 12, more preferably from 6 to 11, still more preferably from 7 to 10, still more preferably about 8 amino acids.

The peptides of the invention may possess linear, branched, cyclic, or constrained backbones. For instance, peptides or functional equivalents with at least two cysteine (C) units can be cyclized by oxidation. If a compound is cyclized via such a disulfide bond, it is preferred that the participating cysteine units are members of the N-and C-terminal groups, respectively. Cyclic structures are, in fact, an example for conformationally constrained backbones. Other types of constrained structures may also be introduced to decrease the conformational flexibility of the compound. Especially the presence of olefinic bonds or small ring structures in the backbone serve this purpose. Examples of such constraints are given in Pharmaceutical Biotechnology, Ed. D. J. A. Crommelin and R. D. Sindelar, Harwood Academic Publishers, 1997, p. 139f.

The invention provides methods for the preparation of such a peptide according to the invention. Such methods include the chemical and enzymatic ligation of amino acids monomers or oligomers to assemble the peptides. They also include the expression of nucleic acid sequences encoding the compounds in host cells, using a vector for transfecting the host cells with the nucleic acid sequences, and obtaining the peptides from the host cell. The poresent invention thus also relates to the nucleic acid sequence, encoding the peptides of the invention.

The peptides of the invention may be used alone, or in combination in the form of multimers. Suitable combinations of peptides of the invention comprise concatemers of peptides of the invention serially coupled to each other via spacers, for instance in the form of a peptide dimer, a peptide trimer, etc., wherein the individual peptides are subsequently aligned. Single peptide or peptoid chains may be coupled to a biocompatible protein, such as human serum albumin, humanized antibody, liposome, micelle, synthetic polymer, nanoparticle, and phage. Alternatively, multimers of individually combined peptides of the invention may be prepared in the form of dendrimers, or clusters, wherein three or more peptides are linked to one common centre. In fact, a dendrimer comprising three peptides according to the invention that are linked together at one common centre may in certain aspects of the present invention be preferred since this form is most complementary to the collagen-like binding site of the SR-A receptor.

Yet other combinations in the form of multimers may be formed by beads on the surface of which the peptides of the invention are exposed. The bead may then function as a carrier for the peptide, and may similarly function as a detectable label. Multimers can, for example, be prepared by biotinylating the N-terminus of peptide or peptoid chains and subsequent complexation with streptavidin. As streptavidin is able to bind 4 biotin molecules or conjugates with high affinity, very stable tetrameric peptide complexes can be formed by this method. Multimers may be composed of identical or different peptides or functional equivalents. Preferably, however, the multimers of the invention are composed of two or more identical peptides or functional equivalents.

The peptides can generally be prepared by the methods that are known for the preparation of peptides and similar substances. Smaller peptides containing only a few amino acids or similar units, and preferably not more than 30-50 units, can be prepared by chemical or enzymatic ligation techniques, either using the classical approach in which the reactions take place in solution or suspension, or by employing a solid phase approach, in which the peptide is assembled while being anchored to a solid surface, such as a polymeric bead. Larger compounds are typically synthesized by automatic solid phase peptide synthesizers.

Alternatively, the peptides can be prepared by known genetic engineering techniques. This approach is especially valid if the compound is indeed a peptide or a slightly modified peptide. For instance, a nucleotide sequence which encodes the peptide can be associated or combined with an expression vector capable of transfecting cells. In another step of the method, host cells or target cells are transfected with said nucleotide sequence, e.g. in the form of DNA, by contacting the cells with the vector and the vector-associated nucleotide sequence under conditions which allow transfection. In a further step, the host or target cells are cultured under conditions which allow the expression of the peptide. Subsequently, the peptide can be isolated. If the peptide itself cannot be encoded or expressed but is very similar to a peptide that can be encoded or expressed, the method can be applied to prepare the peptide to which the peptide is similar, followed by one or more steps in which the peptide is modified by chemical or enzymatic techniques to prepare the peptide.

Various types of vectors are used for this purpose, such as viral vectors, lipoplexes, polyplexes, microspheres, nanospheres, dendrimers, naked DNA, peptide delivery systems, lipids, especially cationic lipids, or liposomes made thereof, polymeric vectors, especially those made of polycationic polymers. Among the preferred viral vectors are retroviruses, adenoviruses, adeno-associated viruses, herpes simplex viruses, and virosomes. Preferred non-viral vectors include chitosan, SPLP, polymeric systems based on PLGA, polyethyleneimines, polylysines, polyphosphoamidates, poly(meth)acrylates, polyphosphazenes ; DOPE, DOTAP, and DOTMA.

Some more comprehensive summaries of methods which can be applied in the preparation of the peptides are described in: W. F. Anderson, Nature 392 Supp., 30 April 1998, p. 25-30; Pharmaceutical Biotechnology, Ed. D. J. A. Crommelin and R. D. Sindelar, Harwood Academic Publishers, 1997, p. 53-70, 167-180, 123-152, 8-20; Protein Synthesis: Methods and Protocols, Ed. R. Martin, Humana Press, 1998, p. 1-442; Solid-Phase Peptide Synthesis, Ed. G. B. Fields, Academic Press, 1997, p. 1-780; Amino Acid and Peptide Synthesis, Oxford University Press, 1997, p. 1-89.

Salts of peptides or functional equivalents are prepared by known methods, which typically involve the mixing of the peptide or peptoid with either a pharmaceutically acceptable acid to form an acid addition salt, or with a pharmaceutically acceptable base to form a base addition salt. Whether an acid or a base is pharmaceutically acceptable can be easily decided by a person skilled in the art after taking the specific intended use of the compound into consideration. For instance, not all acids and bases that are acceptable for *in vitro* diagnostic compositions can be used for therapeutic compositions. Depending on the intended use, pharmaceutically acceptable acids include organic and inorganic acids such as formic acid, acetic acid, propionic acid, lactic acid, glycolic acid, oxalic acid, pyruvic acid, succinic acid, maleic acid, malonic acid, cinnamic acid, sulfuric acid, hydrochloric acid, hydrobromic acid, nitric acid, perchloric acid, phosphoric acid, and thiocyanic acid, which form ammonium salts with free amino groups of peptides and functional equivalents. Pharmaceutically acceptable bases, which form carboxylate salts with free carboxylic groups of peptides and functional equivalents, include ethylamine, methylamine, dimethylamine, triethylamine, isopropylamine, diisopropylamine, and other mono-, di- and trialkylamines, as well as arylamines. Moreover, also pharmaceutically acceptable solvates are encompassed.

The utility of the peptides of the invention as described above is divers. First and foremost, the peptides may be used as a medicament. As described, antagonists of SR-A receptors may reduce the deleterious effect of SR-A on atherosclerotic lesion formation and atherogenesis. Thus, the peptides of the invention may be used as medicament for the treatment or prevention of atherosclerosis.

Further, the peptides of the present invention may be used for targeted delivery of molecules, preferably drugs. The peptides of the invention may provide a molecular targeting system which may be chemically fused to a chosen molecule. The peptides of the present invention are in a preferred embodiment capable of targeting drugs to cell surfaces that exhibit the ligand binding site of the SR-A receptor, thus effectively 'locking' the drug where it is needed. The peptides of the present invention may demonstrate site-specific targeting and allow site specific targeting and retention of systemically administered therapeutics. Due to their high selectivity for binding to the scavenging receptor, they may used to deliver pharmaceutical compounds to the SR-A receptor in particular, or to SR-A receptor-dense localities within the human or animal body, which pharmaceutical compounds may for instance be conjugated to the peptide of the invention in position X₁ or X₄ in formula I. This type of delivery has the advantages that an increased efficacy at the site of action can be attained and that it will result in reduced circulating drug concentrations. In order to function as drug delivery vehicles, the peptides of the present invention may be conjugated to suitable drugs, by any method available to those skilled in the art. The peptide-drug conjugates may then be administered to the human or animal body or may be further processed by know methods. Thus, the peptides of the invention may be conjugated to a known medicament for treating atherosclerosis or for treating atherosclerotic lesions, and the thus obtained conjugate may be administered to the human or animal body in order to deliver the medicament for treating atherosclerosis to the SR-A receptor, or the SR-A receptor-dense localities within the human or animal body. In that case, the peptide-conjugate itself is used as a medicament.

Suitable drugs or medicaments that may be conjugated to the peptides of the invention may include for instance anti inflammatory drugs, including glucocorticoids and non-steroidal anti inflammatory drugs (NSAIDs); proteasome inhibitors for cancer therapy such as, MG 132, PSI, lactacystin, or epoxomicin; immunosuppressive drugs including calcineurin inhibitors such as cyclosporin A, FK506 and inhibitors of transcription factors such as nuclear factor of activated T cells (NFAT) Nuclear Factor kappa-B (NFkB) inhibitors as well as transcription factor decoys; small interfering RNA; and antisense drugs.

For instance, the peptides of the invention may be conjugated to an anti-inflammatory drug in order to target such a drug to the SR-A receptor where it can exert its beneficial effect.

In yet another alternative embodiment, a peptide-conjugate according to the invention may be prepared by combining the peptide of the invention, or a peptide-conjugate of the invention, with a detectable label. Such a labeled peptide or peptide-conjugate may be used for targeted imaging and diagnostics of rupture-sensitive plaques. The peptide-label conjugate is preferably prepared such that it is suitable for administration to the human or animal body, e.g. is preferably prepared such that it is not toxic. Upon migration of the peptide-label conjugate through the body it will accumulate at the ligand binding site of the SR-A receptor. The subsequent detection or localization of the detectable label thereby identifies the localities that are rich in SR-A receptors. Such localities are potential atherosclerotic lesions. Peptide conjugates of the invention are thus advantageously used in targeted imaging applications. Suitable labels for such applications include contrast agents, such as the DTPA (diethylene-triaminepenta acetic acid)-gadolinium (Gd³⁺) complexes for magnetic resonance imaging, or those agents described in United States Patent Application 20030185760, to Lanza *et al*., and references disclosed therein; nanoparticles, such as ultrasmall super paramagnetic iron oxide (USPIO) nanoparticles, ¹⁹F rich compounds or radiolabels (bv. ^{99m}Tc, ¹³³I), or attachment to macroscopic carriers for these agents such as liposomes and polymers nanoparticles. An advantage of the peptides of the present invention over antibodies in targeted imaging techniques is that coupling to nanoparticles is much easier with peptides and that these can be prepared at greater purity. In both these aspects, the application of defined sequences has advantages over the use of antibodies.

The detection (and/or localization) of the peptide conjugates according to the invention may in principle occur *in vivo , ex vivo* and/or *in vitro.* Such detection methods are widely used in all fields of medicine, specifically those relating radiology and imaging in internal medicine, pathology and cardiology. The skilled person will appreciate the various possibilities for, for instance, staining tissue sections with the peptide conjugates of the invention and detecting the peptide conjugates of the invention therein. Such procedures will essentially be those used in immunohistochemical staining. Alternatively detection of the peptide conjugates according to the invention may occur *in vivo* and may then be performed by using suitable techniques such as MRI, X-ray imaging and relating modalities optionally performed on the whole body of an individual. The skilled person will be understand that the method of detection should be chosen such that they are compatible with the labels conjugated and the specific application.

Thus, the peptides of the present invention may target drugs, labels or other molecules to the SR-A thus localizing these drugs, labels or other molecules at the SR-A.

In a method for producing a medicament or pharmaceutical composition according to the invention, a plurality of different peptides is produced, preferably by using a phage display library as described, and the binding of these peptides to the ligand binding site of SR-A is tested. Subsequently, a peptide which binds to said ligand binding site of SR-A is selected and a medicament or pharmaceutical composition comprising the thus selected peptide is manufactured by methods known to the skilled person.

A pharmaceutical composition of the invention comprises a therapeutically effective amount of one or more peptides of the present invention. Once formulated, the pharmaceutical compositions of the invention can be administered directly to the subject in a method of treating atherosclerosis comprising administering to a subject in need thereof a therapeutically effective amount of the composition of the invention.

Direct delivery of the compositions will generally be accomplished by parenteral injection or other forms of administration, either orally, topically, subcutaneously, sublingually, intralesionally, intraperitoneally, intravenously or intramuscularly, pulmonarily, or delivered to the interstitial space of a tissue.

The pharmaceutical composition may also comprise a suitable pharmaceutically acceptable carrier or diluent and may be in the form of a capsule, tablet, lozenge, dragee, pill, droplet, suppository, powder, spray, vaccine, ointment, paste, cream, inhalant, patch, aerosol, and the like. As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the peptide or peptide conjugate. It may be preferred to further include an adjuvant in the dosage form, in order to further treat or prevent the atherosclerogenesis.

A pharmaceutical composition may also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

For therapeutic treatment, peptide or peptide-conjugate may be produced as described above and applied to the subject in need thereof. The peptide or peptide-conjugate may be administered to a subject by any suitable route, preferably in the form of a pharmaceutical composition adapted to such a route and in a dosage which is effective for the intended treatment. Therapeutically effective dosages of the peptide or peptide-conjugate required for treating the SR-A related disorder, for instance for decreasing the atherogenic action of high SR-A activity in the body of a human or animal subject, can easily be determined by the skilled person, for instance by using animal models.

The term "therapeutically effective amount" as used herein refers to an amount of a therapeutic, *viz*. a peptide or peptide-conjugate according to the present invention, to reduce or prevent formation of atherosclerotic lesion, or to exhibit a detectable therapeutic or prophylactic effect. The effect can be detected by, for example, imaging of atherosclerotic lesions as described herein or by any other suitable method of assessing the progress or severity of atherosclerosis. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician or experimenter. Specifically, the compositions of the present invention can be used to reduce or prevent atherosclerosis and/or inflammation and/or accompanying biological or physical manifestations, such as attenuation of pain or the attenuation of interleukin levels in blood. Methods that permit the clinician to establish initial dosages are known in the art. The dosages determined to be administered must be safe and efficacious.

For purposes of the present invention, an effective dose will be from about 0.01 µg/kg to 1 mg/kg or 0.5 µg/kg to about 100 µg/kg of the peptide or peptide-conjugate in the individual to which it is administered.

Yet in another alternative embodiment, the peptide or peptide-conjugate or compositions of the invention may be administered from a controlled or sustained release matrix inserted in the body of the subject.

It may also be advantageous to administer a compound of the invention in a transmucosal dosage form. This route of administration is non-invasive and patient-friendly; at the same time it may lead to an improved bioavailability of the compound compared to oral administration, especially if the compound is not stable in the fluids of the digestive system, or if it is too large to be absorbed from the gut effectively. Transmucosal administration is possible, for instance, via nasal, buccal, sublingual, gingival, or vaginal dosage forms. These dosage forms can be prepared by known techniques; they can be formulated to represent nasal drops or sprays, inserts, films, patches, gels, ointments, or tablets. Preferably, the excipients used for a transmucosal dosage form include one or more substances providing for mucoadhesion, thus prolonging the contact time of the dosage form with the site of absorption and thereby potentially increasing the extent of absorption.

In a further embodiment, the compounds are administered via the pulmonary route, using a metered dose inhaler, a nebulizer, an aerosol spray, or a dry powder inhaler. Appropriate formulations can be prepared by known methods and techniques. Transdermal, rectal, or ocular administration may also be feasible in some cases.

It can be advantageous to use advanced drug delivery or targeting methods to deliver a compound of the invention more effectively. For instance, if a non-parenteral route of administration is chosen, an appropriate dosage form may contain a bioavailability enhancing agent, which may be any substance or mixture of substances which increases the availability of the compound. This may be achieved, for instance, by the protection of the compound from degradation, such as by an enzyme inhibitor or an antioxidant. More preferably, the enhancing agent increases the bioavailability of the compound by increasing the permeability of the absorption barrier, which is typically a mucosa. Permeation enhancers can act via various mechanisms; some increase the fluidity of mucosal membranes, while others open or widen the gap junctions between mucosal cells. Still others reduce the viscosity of the mucus covering the mucosal cell layer. Among the preferred bioavailability enhancers are amphiphilic substances such as cholic acid derivatives, phospholipids, ethanol, fatty acids, oleic acid, fatty acid derivatives, EDTA, carbomers, polycarbophil, and chitosan.

The present invention also provides a method of imaging. An *in vivo* method of imaging atherosclerotic lesions or diagnosing rupture-sensitive atherosclerotic plaques according to the invention comprises the step of administering to the body of a subject in need thereof a peptide-conjugate according to the invention wherein said conjugate comprises a label, followed by the step of *in vivo* detecting the location and/or density of said peptide-conjugate in said body. See for such methods of detecting for instance Jaffer and Weissleder, 2004; Dancey, 2003; Jacoby et al., 2004; Artemov, 2003; Li and Bednarski, 2002. The localization of the peptide-conjugate in the body will enable the determination of the position where atherosclerotic lesions or diagnosing rupture-sensitive atherosclerotic plaques are localized.

In another embodiment, the method of imaging atherosclerotic lesions or diagnosing rupture-sensitive atherosclerotic plaques may be performed on a sample of a body, preferably a tissue sample such as a cardiovascular tissue sample, obtained from the body of a subject, administering to said sample a peptide-conjugate according to the invention wherein said conjugate comprises a label, followed by the step of detecting the position and/or density of said peptide-conjugate in said sample.

The invention will now be illustrated by way of the following, nonlimiting examples.

### EXAMPLES

### Materials and Methods

### Phage library

The pComb3 phage displayed peptide library X₁₅ (peptide [length 15 aa] in which X is any amino acid) was generated by Dr Pannekoek and coworkers (University of Amsterdam, the Netherlands) (Gardsvoll *et al*., 1998).

### Peptide synthesis

All peptides were synthesized on an automated peptide synthesizer (9050 Millipore, MA) using standard Fmoc solid-phase peptide synthesis. Crude peptides were purified on a preparative C8 RP-HPLC column (Altech, Deerfield, IL) using a JASCO PU-980 (Tokyo, Japan). Purified peptides were further characterized by LC-MS in Division of Analytical Biosciences, Leiden University. The purity of the peptide, as checked by MALDI-TOF mass spectrometry and RP-HPLC, was at least 70%. Lyophilized peptides were stored at -20°C under nitrogen until further use. Synthetic biotinylated bovine SR-AI was synthesized, purified and characterised as described by Suzuki et al. (Suzuki *et al*., 1999).

### Selection of SR-AI binding phage

10 µg/ml of streptavidin in coating buffer (50 mM NaHC03, pH 9.6) was incubated overnight at 4°C in a high binding 96 well plate (Costar, Coming, UK) at 100 µl/well. Subsequently, wells were washed with assay buffer (20 mM HEPES, 150 mM NaCl, 1mM CaCl₂, pH 7.4) and incubated for 1 hour at 37°C with blocking buffer (3% BSA in assay buffer). After washing, wells were incubated with 50 µM of biotinylated SR-AI (2 h 37°C), washed, and subsequently incubated for 2 hours at room temperature (RT) with the phage libraries at 10⁹ colony forming units (CFU) in 100 µl of binding buffer (0.1% BSA, 0.5% Tween 20 in assay buffer). Wells were washed 10x with binding buffer and binding phage were eluted by incubation for 5 min at RT with 100 µl of elution buffer (0.1 M glycine/HCI, pH 2.2) and neutralized by addition of 50 µl of neutralization buffer (1M Tris/HCI, pH 8.5). Phage were titrated, amplified and purified as described (Molenaar *et al*., 2002). Amplified phage was used for further selection rounds. For DNA sequencing of enriched phage pools, plasmid DNA was isolated from single colonies using the Wizard plus SV Miniprep DNA Purification System (Promega, Madison, USA). DNA sequencing of the plasmids was conducted at the DNA-sequencing facility of the Leiden University Medical Center using a standard M13 primer. Unless otherwise stated, the amplified phage pool of round 7 was used for all experiments.

### Cell binding assay

COS-7 and RAW cells were grown to approximately 60-70% confluency in 12 wells plates, while the non-adherent THP-1 cells were grown to 4•10⁵ cells. Cells were pre-incubated in DMEM+0.2% BSA, 2h at 37°C. SR-AI specific phage (round 7) was added at a titre of 10⁸ CFU and incubated for 2h at 4°C. Non-selected phage library was used as a negative control. RAW and COS-7 cells were washed 10x with PBS, 500 µl lysis buffer (Promega, Reporter E397A) was added. The THP-1 cell suspension was washed 10x by adding PBS, subsequent centrifuging for 5 min at 10,000 rpm and resuspending the cell pellet in PBS. Finally, the pellet was suspended in PBS and cells were lysed by addition of 500 µl lysis buffer. Input samples were taken from the incubation medium immediately after addition of the phage and diluted 1:100 in TBS in a total volume of 1 ml. Output samples were taken from the cell lysate and diluted 1:10 or 1:100 (total volume: 500 µl). The recovery of bound phage was calculated from the output to input ratio.

### Competition experiment of SR-AI selected phage pool

In analogy to the phage selection procedure, 10 µg/ml of streptavidin in coating buffer was incubated overnight at 4°C in a high-binding 96-well plate at 100 µl/well. Wells were washed 3x with assay buffer and incubated for 1 h at 37°C with blocking buffer. Next, wells were incubated for 2h at 37°C with 125 nM biotininylated SR-AI binding peptide (100 µl/well), washed 3x with binding buffer, after which the SR-AI ligands or synthetic SR-AI binding peptides (7, 9, 11, 13, 15 a. a.) were added and the solution (100 µl) was left to incubate for 2h at RT with the enriched phage pool (round 7) at 10⁸ cfu. Wells were washed 10x with binding buffer, binding phage were eluted by incubation for 5 min at RT with 100 µl elution buffer, and neutralized with 50 µl neutralization buffer (1M Tris-HCI, pH 8.5). Residual phage binding was calculated from the output ratio.

### RESULTS

### Selection of SR-AI binding phage

A 15 amino acid random phage displayed peptide library was screened for SR-AI binding peptide ligands. To favour the selection of peptides binding to the actual binding pocket of SR-AI, biopanning was performed on wells coated with a streptavidin associated biotinylated peptide encompassing the complete ligand binding motif of SR-A1 (Suzuki *et al*., 1999). In the first two rounds a non-stringent washing protocol with ice-cold binding buffer was used, while from round three onwards stringency was increased by decreasing the coating density of synthetic SR-AI and by using a non-cooled binding buffer for washing. This led to an approximately 500-fold enrichment of binding phage in the 5^{th} round (Fig. IA). Subsequent DNA sequence analysis of 10 phage clones from the 6^{th} and 7^{th} selection round revealed a 12-mer consensus peptide motif. 8/10 phage clones from round 6 shared the peptide sequence LSLERFLRCWSDAPA (PP1; see SEQ ID NO: 1) while 2/10 clones carried a highly homologous LSLERFLRCWSDSPR (PP2; see SEQ ID NO: 2) insert. In 7^{th} round, only PP1 clones were detected suggesting that PP1 phage was more effectively bound than PP2. Compared with the parental non-selected phage library, the enriched phage pool showed high binding to streptavidin immobilized SR-AI peptide but only marginal binding to streptavidin immobilized biotin or to type-2 collagen S. There was no binding of the non-specific phage to streptavidin immobilized SR-AI, confirming that binding of the selected phage pool implicated the SR-AI moiety (Fig. IB).

### Cell binding of the enriched phage pool

To verify whether the selected phage pool also bind to the full-length receptor on cells we have tested the ability of the phage pool binding to human THP-1 and murine RAW cells, which were shown to display SR-AI expression. Compared to the parental phage library, the selected phage specifically bound to human THP-1 cells (Fig.2) and to a much less extent to murine RAW cells, although still somewhat as higher binding as to monkey COS-7 cells, which are deficient in SR-A expression.

### Competition studies of SR-AI binding phage by established SR-AI inhibitors

To further characterize the specificity and the affinity of the selected phage binding, displacement studies of phage binding to SR-AI by the established SR-AI inhibitors poly I and suc-HSA were performed. Both poly I (IC₅₀=10.5 µg/ml) and suc-HSA (IC₅₀=66 µg/ml) could dose-dependently compete for phage binding to SR-AI (Fig.3). DNA sequence analysis revealed the presence of a 12-mer consensus sequence within the peptide insert of the SR-AI binding phage, suggesting the N-terminal part of the phage encoded peptide to be relevant for SR-AI binding. To pinpoint the minimal peptide sequence required for SR-AI binding we have performed a truncation study. At which we focused on the N-terminal of the peptide. Full-length peptide LSLERFLRCWSDAPA (PP1) and truncated peptides LERFLRCWSDAPA (PP1-13), RFLRCWSDAPA (PP1-11), LRCWSDAPA (PP1-9) and CWSDAPA (PP1-7) were synthesized with Fmoc chemistry. The capacity of the peptides to interfere with phage binding to SR-AI was tested in a competition assay at which residual binding of the selected phage to SR-AI was determined in the presence of the synthetic peptides (Fig.4). It appears that PP1 can dose-dependently and potently inhibit SR-AI binding of the enriched phage at an IC₅₀ value of 29 µM. Stepwise truncation of PP1 resulted in a gradual reduction of the affinity of the peptide, at which the 11-mer peptide PP1-11 was identified as the minimal motif of PP1 to bind to SR-AI.

### DISCUSSION

In search of potent and selective peptide binders to SR-AI we have employed phage display on a synthetic SR-AI receptor sequence. Phage display of random peptides has developed into a very useful technology for selection of peptide ligands that bind to diverse targets. The synthetic collagen-like sequence has a comparable structure to the binding site of SR-AI, which facilitates for selection of peptides binding to the natural SR-AI.
SR-AI specific phage clones were isolated which shared an 11-mer consensus peptide motif The C-terminus of the two peptides differed, suggesting that in particular the N-terminal part of the peptide in SR-AI binding may be relevant for receptor binding. Protein BLAST search for short nearly exact matches of the N-terminal heptapeptide sequence of PP 1, LSLERFL (see SEQ ID NO: 4), revealed a highly homology to viruses such as human cytomegalovirus (CMV) and HIV (table 1), which also had been implicated in atherosclerosis (Ngeh *et* *al*., 2002). While SR-AI was found to interact with gram-positive bacteria (Dunne *et al*., 1994), it might be suggested that SR-AI may also be involved in the uptake of CMV and HIV through the LSLERFL motif Further research will be required to elucidate the involvement of SR-AI in cellular uptake of the above infectious agents.

The peptide truncation studies revealed that deletion of up to 4 N-terminal amino acids was quite well tolerated in that the affinity of these truncated peptides for SR-AI was only slightly lowered. Together with the result from the BLAST search, it is tempting to assume that the 5-mer peptide LERFL (see SEQ ID NO: 5) is the minimal motif for SR-AI binding. The SR-AI binding phage appeared to be very specific for SR-AI as it did not bind to polystyrene, collagen, or streptavidin immobilized biotin. Phage binding to SR-AI could be inhibited by two established SR-AI inhibitors, suggesting that phage binding indeed involves in the ligand binding domain of SR-AI.

Previous truncation or site-directed mutagenesis studies on the C-terminus of the SR-AI showed that the collagen-like domain was not only essential for ligand binding but also for maintaining the requisite trimeric structure (Doi *et al*., 1993; Acton *et al*., 1993; Tanaka *et al*., 1993; Tanaka *et al*., 1996). Our competition data showed that poly I could effectively compete with phage binding. As poly I is an effective inhibitor of phage binding to the synthetic trimeric SR-AI peptide, this suggests that the peptide antagonist interacts at the level of the collagen-like binding domain, which is also involved in AcLDL binding.

The SR-AI binding phage not only bound avidly to the synthetic receptor hut also to human THP-1 cells and, to a less extent, to murine RAW cells, which are both known to express high levels of SR-AI. The higher binding to THP-1 cells may be attributable to interspecies differences or to differences in SR-AI expression levels between the two cell lines. As no phage binding was observed in SR-AI deficient COS-7 cells, it is conceivable that the enriched phage clone is a potential candidate for targeted inhibition of SR-AI *in vivo*.

It is established that SR-A plays a credited role in foam cell formation process that underlies atherosclerosis. Various *in vivo* studies show that SR-AI is indeed critical in atherosclerosis. (Li *et al*., 1995; Mietus-Snyder *et al*., 1997; Linton and Fazio, 2001). Recently, Kunjathoor V.V. et al (Kunjathoor *et al*., 2002) have demonstrated that SR-A and CD36 are the "preponderance" receptors in the uptake of modified LDL in macrophages *in vitro.* By contrast, more recently identified scavenger receptors including CD68, LOX-1, SR-PSOX, and SREC, were found to play a minor role in modified LDL uptake by macrophages. As it is assumed that a decreased lipid accumulation in intimal macrophages will be accompanied by reduced atherosclerosis, therefore, inhibiting macrophage scavenger receptors may be a viable therapeutic strategy for atherosclerosis. Further research on a combined inhibition of SR-A and CD36 will be helpful to clarify the role of scavenger receptors in foam formation and test alternative pathway in the pathogenesis of atherosclerosis.

In conclusion, we have successfully employed a novel phage display strategy for the design of SR-AI inhibitors, involving biopanning on a synthetic peptide which encompassed the binding domain of the receptor. This strategy led to the identification of a novel 15-mer linear peptide, PP1, which antagonized SR-AI binding with an IC₅₀ of 50 µg/ml. This is only 5 fold less potent than poly I, one of most potent SR-AI inhibitors. Subsequent truncation studies firmly established the N-terminal part of peptide as the minimal essential motif required for SR-AI binding. This peptide may constitute the starting point for the design of even more potent inhibitors of SR-AI (Appeldoorn *et* al., 2003). And prove useful not only in the development of SR-AI receptor antagonists and anti-atherogenic therapy but also of new imaging and targeting strategies aimed at macrophage enriched sites of inflammation such as atherosclerotic plaques.

### REFERENCES

**Acton S, Resnick D, Freeman M, Ekkel Y, Ashkenas J, Krieger M** (1993) *J. Biol. Chem.* 268,3530-3537.
**Appeldoorn CC, Molenaar TJ, Bonnefoy A, van Leeuwen SH, Vandervoort PA, Hoylaerts MF, Van Berkel TJ, Biessen EA.** (2003) *J Biol. Chem.* 278, 10201-10207.
**Artemov D.** 2003. *J Cell Biochem.* 90(3):518-24.
**Ashkenas J, Penman M, Vasile E, Acton S, Freeman M, Krieger M** (1993) *J. Lipid Res.* 34, 983-1000.
Babaev VR, Gleaves LA, Carter KJ, Suzuki H, Kodama T, Fazio S, Linton MF (2000) *Arterloscler. Thromb. Vasc. Biol.* 20, 2593-2599.
**Bickel PE, Freeman MW** (1992) *J Clin. Invest* 90, 1450-1457.
**Blomhofl R, Eskild W, Berg T** (1984) *Biochem. J.* 218, 81-86.
**Dancey JE.** 2003. *Cancer Biol Ther.* 2(6):601-9.
**Daugherty A, Comicelli JA, Welch K, Sendobry SM, Rateri DL** (1997) *Arterioscler. Thromb. Vasc. Biol.* 17, 2369-2375.
**Doi T, Higashino K, Kurihara Y, Wada Y, Miyazaki T, Nakamura H, Uesugi S, Imanishi T, Kawabe Y, Itakura H** (1993) *J Biol. Chem.* 268, 2126-2133.
**Dunne DW, Resnick D, Greenberg J, Krieger M, Joiner KA** (1994) *Proc. Natl. Acad. Sci.* USA 91, 1863-1867.
**El Khoury J, Thomas CA, Loike JD, Hickman SE, Cao L, Silverstein SC** (1994) *J. Biol. Chem. 269,* 10197-10200.
**Fraser I, Hughes D, Gordon S** (1993) *Nature* 364, 343-346.
**Freeman M, Ashkenas J, Rees DJ, Kingsley DM, Copeland NG, Jenkins NA, Krieger M** (1990) *Proc. Natl. Acad. Sci. US.A* 87, 8810-8814.
**Freeman M, Ekkel Y, Rohrer L, Penman M, Freedman NJ, Chisolm GM, Krieger M** (1991) *Proc. Natl. .Acad. Sci. USA* 88, 4931-4935.
**Gardsvoll H, van Zonneveld A J, Holm A, Eldering E, van Meijer M, Dano K, Pannekoek H.** (1998) *FEBS Lett*. 431, 170-174.
**Geng YJ, Hansson GK** (1995) *Scand. J. Immunol.* 42, 289-296.
**Geng YJ, Holm J, Nygren S, Bruzelius M, Sterome S, Hansson GK** (1995) *Arterioscler. Thromb. Vase. Biol.* 15, 1995-2002.
**Goldstein JL, Ho YK, Basu SK, Brown MS** (1979) *Proc. Natl. Acad. Sci. USA* 76, 333-337.
**Greaves DR, Gough PJ, Gordon S** (1998) *Curr. Opin. Lipidol.* 9, 425-432.
**Greenberg JW, Fischer W, Joiner KA** (1996) *Infect. Immun.* 64, 3318-3325.
**Hampton RY, Golenbock DT, Penman M, Krieger M, Raetz CR.** (1991) *Nature* 352, 342-344.
**Hughes DA, Fraser IP, Gordon S** (1994) *Immunol. Lett. 43,* 7-14.
**Hughes DA, Fraser IP, Gordon S** (1995) *Eur. J Immunol*. 25, 466-473.
**Jacoby DS, Mohler III ER, Rader DJ.** 2004. *Curr Atheroscler Rep.* 6(1):20-6.
**Jaffer FA, Weissleder R.** 2004 *Circ Res.* 94(4):433-45.
**Kodama T, Freeman M, Rohrer L, Zabrecky J, Matsudaira P, Krieger M** (1990) *Nature* 343, 531-535.
**Krieger M** (1997) *Curr. Opin. Lipidol.* 8, 275-280.
**Kunjathoor W, Febbraio M, Podrez EA, Moore KJ, Andersson L, Koehn S, Rhee JS, Silverstein R, Hoff HF, Freeman MW.** (2002) *J Biol. Chem*. 277, 49982-49988.
**Li KC, Bednarski MD.** 2002. *J Magn Reson Imaging.* 16(4):388-93.
**Li H, Freeman MW, Libby P** (1995) *J. Clin. Invest* 95, 122-133.
**Linton MF, Fazio S** (2001) *Curr. Opin. Lipidol.* 12, 489-495.
**Luoma J, Hiltunen T, Sarkioja T, Moestrup SK, Gliemann J, Kodama T, Nikkari T, Yla-Herttuala** S (1994) *J. Clin. Invest* 93, 2014-2021.
**Lysko PG, Weinstock J, Webb CL, Brawner ME, Elshourbagy NA.** (1999) *J Pharmacol. Exp. Ther.* 289, 1277-1285.
**Matsumoto A, Naito M, Itakura H, Ikemoto S, Asaoka H, Hayakawa I, Kanamori H, Aburatani H, Takaku F, Suzuki H,** ***et al*****.** (1990) *Proc. Natl. Acad. Sci. U.S.A* 87, 9133-9137.
**Mietus-Snyder M, Friera A, Glass CK, Pitas RE** (1997) *Arterioscler. Thromb. Vasc. Biol.* 17, 969-978.
**Molenaar TJ, Michon I, de Haas S A, van Berkel T J, Kuiper J, Biessen E A.** (2002) *Virology* 293, 182-191.
**Nagelkerke J F, Barto KP, Van Berkel TJ** (1983) *J Biol. Chem.* 258, 12221-12227.
**Naito M, Kodama T, Matsumoto A, Doi T, Takahashi K** (1991) *Am. J Pathol.* 139, 1411-1423.
**Naito M, Suzuki H, Mori T, Matsumoto A, Kodama T, Takahashi K** (1992) Am. *J Pathol.* 141, 591-599.
**Ngeh J, Anand V, Gupta S.** (2002) *Clin. Microbiol Infect.* 8, 2-13.
**Nishikawa K, Arai H, Inoue K** (1990) *J. Biol Chem*. 265, 5226-5231.
**Pearson AM** (1996) C*urr. Opin. Immunol*. 8, 20-28.
**Pitas RE (1990)** *J. Biol. Chem.* 265, 12722-12727.
**Resnick D, Freedman NJ, Xu S, Krieger M** (1993) *J Biol. Chem.* 268, 3538-3545.
**Rohrer L, Freeman M, Kodama T, Penman M, Krieger M** (1990) *Nature* 343, 570-572.
**Suzuki K, Yamada T, Tanaka T.** (1999) *Biochemistry* 38, 1751-1756.
**Suzuki H, Kurihara Y, Takeya M, Kamada N, Kataoka M, Jishage K, Ueda O, Sakaguchi H, Higashi T, Suzuki T, Takashima Y, Kawabe Y, Cynshi O, Wada Y, Honda M, Kurihara H, Aburatani H, Doi T, Matsumoto A, Azuma S, Noda T, Toyoda Y, Itakura H, Yazaki Y, Kodama T,** ***et al*****.** (1997) *Nature* 386, 292-296.
**Tanaka T, Nishikawa A, Tanaka Y, Nakamura H, Kodama T, Imanishi T, Doi T** (1996) *Protein Eng* 9, 307-313.
**Tanaka T, Wada Y, Nakamura H, Doi T, Imanishi T, Kodama T** (1993) *FEBS Lett.* 334,272-276.
**Terpstra V, van Amersfoort ES, van Velzen AG, Kuiper J, Van Berkel TJ** (2000) *Arterioscler. Thromb.Vasc. Biol.* 20, 1860-1872.
**Van Berkel TJ, Van Velzen A, Kruijt JK, Suzuki H, Kodama T** (1998) *Biochem. J.* 331 (Pt 1), 29-35.
**van Velzen AG, Suzuki H, Kodama T, Van Berkel TJ** (1999) *Exp. Cell Res.* 250, 264-271.
**Yla-Herttuala S, Rosenfeld ME, Parthasarathy S, Sigal E, Sarkioja T, Witztum JL, Steinberg D** (1991) *J.Clin.lnvest* 87, 1146-1152.
**Yokota T, Ehlin-Henriksson B, Hansson GK.** (1998) *Exp. Cell Res.* 239, 16-22.
**Zhang H, Yang Y, Steinbrecher UP** (1993) *J. Biol. Chem.* 268, 5535-5542.

## Claims

1. Method for obtaining an antagonist of scavenger receptor SR-A, said method comprising providing a peptide, testing binding of said peptide to the ligand binding site of scavenger receptor SR-A and selecting said peptide in case said peptide is capable of binding to said ligand binding site of SR-A, thereby obtaining the antagonist.

2. Method according to claim 1, wherein said scavenger receptor SR-A is the SR-AI receptor.

3. Method according to claim 1 or 2, wherein said ligand binding site is a synthetic ligand binding site.

4. Method according to claim, wherein said ligand binding site encompassing the C-terminal Lys cluster (320-340) of the collagen-like domain of the macrophage scavenger receptor, preferably in the form of the triple-helical domain.

5. Method according to any one of the preceding claims, wherein said peptide is provided in the form of a phage display library.

6. Peptide obtainable by a method according to any one of the preceding claims.

7. Peptide capable of binding to the ligand binding site of an SR-A receptor.

8. Peptide according to claim 7, wherein said SR-A receptor is the SR-AI receptor.

9. Peptide according to claim 7 or 8, wherein said ligand binding site is a synthetic ligand binding site.

10. Peptide according to any one of claims 6-9 comprising a sequence according to the formula (I):
X₁-X₂-RFLRCWSD-X₃-P-X₄
in which
X₁ is optionally present and may be a carboxylic acid; or one or more D- or L-amino acids or analogues or mimetics thereof; or an anti-inflammatory drug; or a medicament for treating atherosclerosis; or a detectable label;
X₂ is an amino acid or a peptide, preferably X₂ is selected from the group consisting of the peptide (Leu or Ala or Ile)-Ser-(Leu or Ala or Ile)-(Glu or Asp), the peptide Ser-(Leu or Ala or Ile)-(Glu or Asp), the peptide (Leu or Ala or Ile)-(Glu or Asp), and the amino acids Glu and Asp;
X₃ is an amino acid, preferably selected from Ala, Ser, Leu, Ile, Cys and Thr, more preferably selected from Ala and Ser;
X₄ is optionally present and may be a D- or L-amino acid, preferably selected from Ala, Ser, Leu, Ile, Cys and Thr, more preferably selected from Ala and Ser, or an analogue or mimetic thereof; or an anti-inflammatory drug; or a medicament for treating atherosclerosis; or a detectable label, and R, F, L, C, W, S, D and P are the symbols indicating the amino acids Arg, Phe, Leu, Cys, Trp, Ser, Asp and Pro, respectively, according to IUPAC nomenclature and symbolism for amino acids.

11. Peptide according to any one of claims 6-10 comprising a sequence selected from the group consisting of SEQ ID NO: 1, SEQ ID NO: 2, SEQ ID NO: 3, SEQ ID NO: 4 and SEQ ID NO: 5.

12. Peptide according to any one of claims 6-11, essentially consisting of a sequence selected from the group consisting of the peptides according to the formula (I) X₁-X₂-RFLRCWSD-X₃-P-X₄ as defined in claim 12; the peptides of SEQ ID NO: 3 and truncated forms thereof; and derivatives of these peptides.

13. Peptide according to any one of claims 6-12, conjugated to an anti-inflammatory drug, a medicament for treating atherosclerosis or a detectable label.

14. A peptide according to any one of claims 6-12 or a peptide-conjugate according to claim 13, for use as a medicament.

15. Use of a peptide according to any one of claims 6-12 or a peptide-conjugate according to claim 13, for the manufacture of a medicament for the treatment of atherosclerosis and/or inflammation.

16. Use of a peptide according to any one of claims 6-12, as an SR-A antagonist.

17. Use of a peptide according to any one of claims 6-12 or a peptide-conjugate according to claim 13, as a drug delivery vehicle.

18. Use of a peptide according to any one of claims 6-12 or a peptide-conjugate according to claim 13, in a method for targeted imaging of atherosclerotic lesions.

19. Pharmaceutical composition comprising a peptide according to any one of claims 6-12 or a peptide-conjugate according to claim 13 and an pharmaceutically acceptable carrier.

20. The composition of claim 19, wherein said composition is in the form of an intravenously injectable fluid.

21. An *in vivo* method of imaging atherosclerotic lesions or diagnosing rupture-sensitive atherosclerotic plaques, comprising the step of administering to the body of a subject in need thereof a peptide-conjugate according claim 13 wherein said conjugate comprises a label, followed by the step of *in vivo* detecting the location and/or density of said peptide-conjugate in said body.

22. Method for producing a medicament or pharmaceutical composition, comprising providing a peptide, testing binding of said peptide to the ligand binding site of scavenger receptor SR-A, selecting said peptide in case said peptide is capable of binding to said ligand binding site of SR-A and manufacturing a medicament or pharmaceutical composition comprising the thus selected peptide.

23. A method according to claim 22, wherein the medicament or pharmaceutical composition is for the treatment of atherosclerosis.

24. A method according to claim 22 or 23, wherein the medicament or pharmaceutical composition comprises the selected peptide together with a pharmaceutically acceptable carrier or diluent.

25. A method according to any one of claims 22-24, wherein said peptide is conjugated to an anti-atherosclerotic drug.

26. Pharmaceutical composition obtainable by a method according to any one of claims 22-25.

27. Method of treating atherosclerosis comprising administering to a subject in need thereof a therapeutically effective amount of the composition of claim 19, 20 or 26.


SEQUENCE LISTING

<110> Universiteit Leiden

<120> SR-A antagonists

<130> P68106EP00

<140> 04076659.4
<141> 2004-06-03

<160> 12

<170> PatentIn Ver. 2.1

<210> 1
<211> 13
<212> PRT
<213> Artificial Sequence

<220>
<223> Description of Artificial Sequence: peptide
      binding to scavenger receptor SR-A binding pocket

<220>
<223> /note="Sequence wherein X on position 1 is optionally
      present and may be a carboxylic acid; or one or more
      D- or L-amino acids or analogues or mimetics thereof;
      or an anti-inflammatory drug; or a medicament for treating
      atherosclerosis; or a detectable label.
      X on position 2 is a D- or L-amino acid or a peptide,
      preferably selected from the group consisting of
      the peptide (Leu or Ala or Ile)-Ser (Leu or Ala
      or Ile)-(Glu or Asp), the peptide Ser-(Leu or Ala
      or Ile)-(Glu or Asp), the peptide (Leu or Ala or Ile)
      -(Glu or Asp), and of the amino acids Glu and Asp.
      X on position 11 is a D- or L-amino acid, preferably
      selected from Ala, Ser, Leu, Ile, Cys and Thr, more
      preferably selected from Ala and Ser.
      X on position 13 is optionally present and may be a
      D-or L-amino acid, preferably selected from Ala Ser,
      Leu, Ile, Cys and Thr, more preferably selected from
      Ala and Ser, or an analogue or mimetic thereof; or
      an anti-inflammatory drug; or a medicament for treating
      atherosclerosis; or a detectable label"

<400> 1
Xaa Xaa Arg Phe Leu Arg Cys Trp Ser Asp Xaa Pro Xaa
  1               5                  10


<210> 2
<211> 15
<212> PRT
<213> Artificial Sequence

<220>
<223> Description of Artificial Sequence: peptide
      binding to scavenger receptor SR-A binding pocket

<400> 2

Leu Ser Leu Glu Arg Phe Leu Arg Cys Trp Ser Asp Ala Pro Ala
1               5                   10                  15

<210> 3
<211> 13
<212> PRT
<213> Artificial Sequence

<220>
<223> Description of Artificial Sequence: peptide
      binding to scavenger receptor SR-A binding pocket

<400> 3
Leu Glu Arg Phe Leu Arg Cys Trp Ser Asp Ala Pro Ala
1               5                   10

<210> 4
<211> 11
<212> PRT
<213> Artificial Sequence

<220>
<223> Description of Artificial Sequence: peptide
      binding to scavenger receptor SR-A binding pocket

<400> 4
Arg Phe Leu Arg Cys Trp Ser Asp Ala Pro Ala
1               5                   10

<210> 5
<211> 9
<212> PRT
<213> Artificial Sequence

<220>
<223> Description of Artificial Sequence: peptide
      binding to scavenger receptor SR-A binding pocket

<400> 5
Leu Arg Cys Trp Ser Asp Ala Pro Ala
1               5

<210> 6
<211> 7
<212> PRT
<213> Artificial Sequence

<220>
<223> Description of Artificial Sequence: peptide
      binding to scavenger receptor SR-A binding pocket

<400> 6
Cys Trp Ser Asp Ala Pro Ala
1               5

<210> 7

<211> 4
<212> PRT
<213> Artificial Sequence

<220>
<223> Description of Artificial Sequence: peptide
      binding to scavenger receptor SR-A binding pocket

<400> 7
Leu Ser Leu Glu
  1


<210> 8
<211> 15
<212> PRT
<213> Artificial Sequence

<220>
<223> Description of Artificial Sequence: peptide
      binding to scavenger receptor SR-A binding pocket

<400> 8
Leu Ser Leu Glu Arg Phe Leu Arg Cys Trp Ser Asp Ser Pro Arg
  1               5                  10                  15


<210> 9
<211> 12
<212> PRT
<213> Artificial Sequence

<220>
<223> Description of Artificial Sequence: peptide
      binding to scavenger receptor SR-A binding pocket

<400> 9
Leu Ser Leu Glu Arg Phe Leu Arg Cys Trp Ser Asp
  1               5                  10


<210> 10
<211> 7
<212> PRT
<213> Artificial Sequence

<220>
<223> Description of Artificial Sequence: peptide
      binding to scavenger receptor SR-A binding pocket

<400> 10
Leu Ser Leu Glu Arg Phe Leu
  1               5


<210> 11
<211> 5
<212> PRT
<213> Artificial Sequence

<220>

<223> Description of Artificial Sequence: peptide
      binding to scavenger receptor SR-A binding pocket

<400> 11
Leu Glu Arg Phe Leu
  1               5


<210> 12
<211> 12
<212> PRT
<213> Artificial Sequence

<220>
<223> Description of Artificial Sequence: peptide
      binding to scavenger receptor SR-A binding pocket

<220>
<223> /note="Sequence wherein X on position 1 is optionally
      present and may be a carboxylic acid; or one or more
      D- or L-amino acids or analogues or mimetics thereof;
      or an anti-inflammatory drug; or a medicament for treating
      atherosclerosis; or a detectable label.
      X on position 2 is a D- or L-amino acid or a peptide,
      preferably selected from the group consisting of
      the peptide (Leu or Ala or Ile)-Ser (Leu or Ala
      or Ile)-(Glu or Asp), the peptide Ser-(Leu or Ala
      or Ile)-(Glu or Asp), the peptide (Leu or Ala or Ile)
      -(Glu or Asp), and of the amino acids Glu and Asp.
      X on position 10 is a D- or L-amino acid, preferably
      selected from Ala, Ser, Leu, Ile, Cys and Thr, more
      preferably selected from Ala and Ser.
      X on position 12 is optionally present and may be a
      D-or L-amino acid, preferably selected from Ala Ser,
      Leu, Ile, Cys and Thr, more preferably selected from
      Ala and Ser, or an analogue or mimetic thereof; or
      an anti-inflammatory drug; or a medicament for treating
      atherosclerosis; or a detectable label"

<400> 12
Xaa Xaa Arg Phe Leu Arg Cys Trp Ser Xaa Pro Xaa
  1               5                   10